**Europäisches Patentamt**

**European Patent Office**

**Office européen des brevets**

(19)

(11) Veröffentlichungsnummer: **0 125 495**
**A1**

(12) # EUROPÄISCHE PATENTANMELDUNG

(21) Anmeldenummer: **84104059.5**

(22) Anmeldetag: **11.04.84**

(51) Int. Cl.³: **A 61 N 1/40**

(30) Priorität: **12.04.83 DE 8310561 U**

(43) Veröffentlichungstag der Anmeldung:
**21.11.84 Patentblatt 84/47**

(84) Benannte Vertragsstaaten:
**AT BE CH DE FR GB IT LI NL SE**

(71) Anmelder: **Kief, Horst, Dr.**
**Londoner Ring 105-107**
**D-6700 Ludwigshafen(DE)**

(72) Erfinder: **Kief, Horst, Dr.**
**Londoner Ring 105-107**
**D-6700 Ludwigshafen(DE)**

(54) **Gerät zur Ganzkörperbehandlung mittels elektrischer Felder.**

(57) Vorrichtung zur therapeutischen Behandlung des menschlichen Körpers mittels eins elektrischen Feldes, welches durch einen elektrischen Strom erzeugt wird, der eine Elektrolytlösung (1) durchfließt, die in einer kissenähnlichen oder matratzenähnlichen Kunststoffumhüllung (2) gehalten wird, wobei innerhalb der letzteren Elektroden (3, 4) angeordnet sein können.

Fig. 2

EP 0 125 495 A1

**0125495**

## Gerät zur Ganzkörperbehandlung mittels elektrischer Felder

Die Beeinflussung des menschlichen Körpers durch hochfrequente Wechselfelder zur Wärmetherapie ist seit Jahrzehnten Standard in der physikalischen Medizin. Relativ neu in der derzeitigen Medizin sind niederfrequente magnetische Felder oder die Anwendung niederfrequenter elektrischer Felder. All diese Applikationen müssen, wenn sie nicht in unförmige Größenordnungen ausarten sollen, auf kleinere Areale und Teile des Körpers beschränkt bleiben. Die vorzuschlagende Neuerung erlaubt es, ohne großen technischen Aufwand den gesamten menschlichen Körper einem großflächigen elektrischen Wechselfeld zu therapeutischen Zwecken zu unterziehen. Erfindungsgemäß wird dies dadurch bewerkstelligt, daß ein mit einer Elektrolytlösung (1) gefüllter Kunststoffbeutel (2), ähnlich den sogenannten "Wasserbetten", in seinem Inneren an beiden Schmalseiten mit Elektroden (3) versehen wird. Über diese Elektroden werden nun elektrische Impulse in den Elektrolyten gegeben, wobei als Impulsgenerator durchaus ein Reizstromgerät herkömmlicher Art mit therapeutisch bereits erprobten Stromimpulsen dienen kann. Der Patient wird auf dem "Wasserbett" gelagert und dabei auf für ihn angenehme Weise elektrotherapeutisch behandelt. Das Verfahren hat nicht nur den Vorteil der einfachen problemlosen Handhabung, der Verwendung bereits vorhandenen Materials - etwa von Reizstromgeräten, deren Impulse im Bedarfsfall lediglich verstärkt werden müssen - sondern auch den der absoluten Sicherheit, da der Patient durch die Kunststoffhülle des "Wasserbettes" vom unmittelbaren elektrischen Kontakt isoliert ist. Dabei kann es durchaus von Vorteil sein, daß die Richtung des angelegten elektrischen Feldes variiert wird, indem man zwei zusätzliche Elektroden (4) an den Längsseiten des "Wasserbettes" anbringt.

0125495

Schutzansprüche

1. Vorrichtung zur therapeutischen Behandlung des menschlichen Körpers mittels eins elektrischen Feldes, dadurch gekennzeichnet, daß dieses Feld durch einen elektrischen Strom erzeugt wird, der eine Elektrolytlösung durchfließt, die in einer kissenähnlichen oder matratzenähnlichen Kunststoffumhüllung gehalten wird.

2. Vorrichtung nach Anspruch 1, dadurch gekennzeichnet, daß innerhalb der Kunststoffumhüllung Elektroden angelegt sind.

3. Vorrichtung nach Anspruch 1 bis 2, dadurch gekennzeichnet, daß die genannten Elektroden vorzugsweise an den Längs- oder Schmalseiten auf der Innenseite der Kunststoffumhüllung angebracht sind.

0125495

Fig. 1

Fig. 2

0125495

EUROPÄISCHER RECHERCHENBERICHT

Nummer der Anmeldung

EP 84 10 4059

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 418 937 (KAMML)<br>* Seite 3, letzter Abschnitt - Seite 5, Zeile 8; Figuren 1,5b * | 1,3 | A 61 N 1/40 |
| Y | US-A-4 094 322 (KATSUMASA)<br>* Spalte 1, Zeilen 58-68; Spalte 4, Zeilen 29-30; Figur 8 * | 1,3 | |
| A | DE-A-2 911 758 (MUTZHAS)<br>* Seite 9, Zeilen 16-27; Figur 1 * | 1 | |
| A | 1981 IEEE MTT-S INTERNATIONAL MICROWAVE SYMPOSIUM DIGEST, 1981, Seiten 463-464, IEEE, New York, USA; R.W. PAGLIONE et al.: "27 MHz waveguide applicators for localized hyperthermia treatment of cancer"<br>* Seite 463, linke Spalte, letzter Abschnitt und rechte Spalte * | 1 | **RECHERCHIERTE SACHGEBIETE (Int. Cl. 3)**<br><br>A 61 N<br>A 47 C |
| A | FR-A- 685 585 (OFFENBACHER)<br>* Seite 1, Zeilen 9-22, 44-46; Seite 2, Zeilen 33-40 * | 1,2 | |
| A | GB-A-1 140 767 (WEINSTEIN)<br>* Seite 3, Zeilen 51-61 * | 1 | |

--- -/-

Der vorliegende Recherchenbericht wurde für alle Patentansprüche erstellt.

| Recherchenort | Abschlußdatum der Recherche | Prüfer |
|---|---|---|
| DEN HAAG | 25-07-1984 | SIMON J.J.E. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-4 233 492 (McMULLAN) <br> * Spalte 2, Zeilen 6-20 * <br><br> ----- | 2 | |

RECHERCHIERTE
SACHGEBIETE (Int. Cl. ³)

Der vorliegende Recherchenbericht wurde für alle Patentansprüche erstellt.

| Recherchenort <br> DEN HAAG | Abschlußdatum der Recherche <br> 25-07-1984 | Prüfer <br> SIMON J.J.E. |
|---|---|---|

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

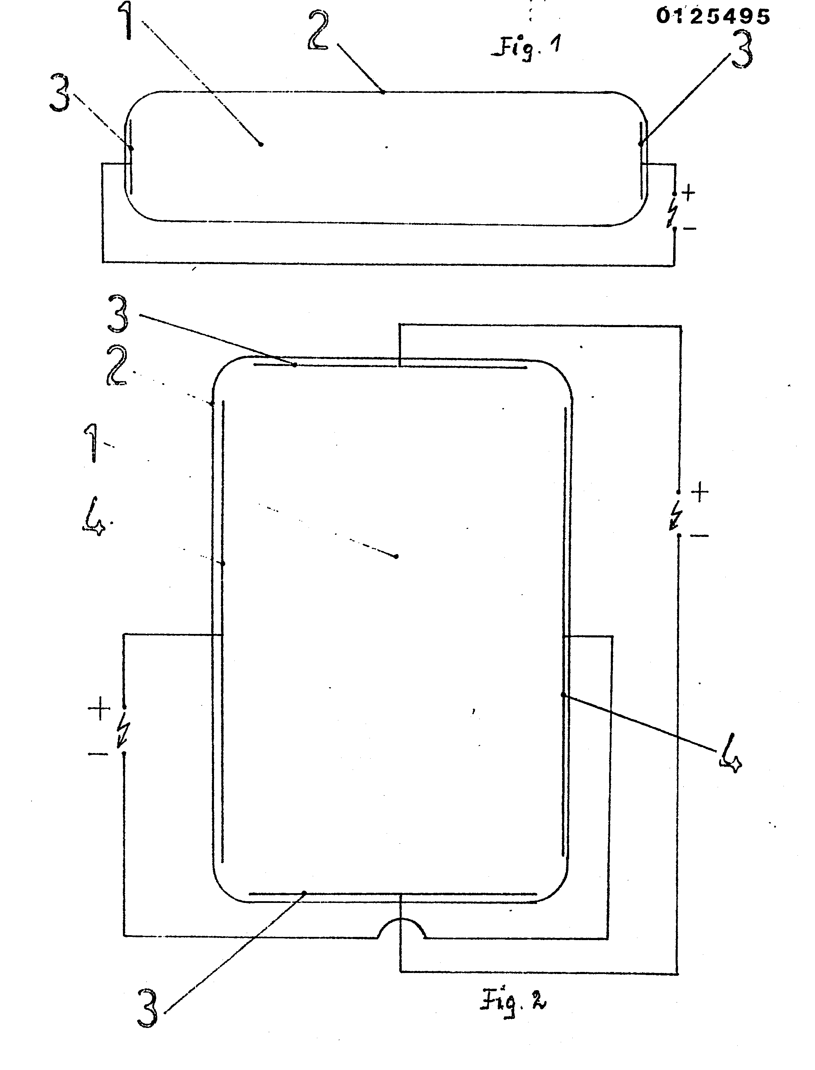

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82